# DEMANDE DE BREVET EUROPEEN

(11) **EP 1 462 093 A2**
(43) Date de publication de la demande: **29.09.2004**
(21) Numéro de dépôt: 04290799.8
(22) Date de dépôt: 25.03.2004
(51) Int. Cl.: A61K 7/13, A61K 7/06

(54) **Composition de coloration pour fibres kératiniques comprenant au moins un acide polycarboxylique ou un sel, composition prête à l'emploi la comprenant, procédé de mise en oeuvre et dispositif**

(30) Priorité: 25.03.2003 FR 0350062; 28.03.2003 FR 0303874
(71) Demandeur: L'OREAL, 75008 Paris (FR)
(72) Inventeur: Desenne, Patricia, 92270 Bois Colombes (FR); Millequant, Jean-Marie, 94100 Saint-Maur des Fosses (FR)
(74) Mandataire: Wattremez, Catherine

(57) **Abrégé**

La présente invention a pour objet une composition, comprenant dans un milieu approprié pour la teinture a) au moins une base d'oxydation et/ou au moins un colorant direct et b) au moins un composé acide polycarboxylique particulier ou un de ses sels.

Elle a de même pour objet une composition prête à l'emploi comprenant la composition précitée et un oxydant.

L'invention concerne de plus un procédé de coloration de fibres kératiniques, dans lequel on applique sur lesdites fibres, une composition selon l'invention et éventuellement une composition oxydante, celles-ci étant mélangées extemporanément avant l'emploi ou appliquées successivement ; on laisse reposer pendant un temps suffisant pour obtenir la coloration recherchée ; on rince les fibres pour en éliminer la composition selon l'invention et éventuellement la composition oxydante ; on lave éventuellement les fibres, on les rince après chaque lavage et éventuellement, on les sèche.

Elle a enfin pour objet un dispositif pour la mise en oeuvre du procédé.

## Description

La présente invention a pour objet une composition destinée à la coloration des fibres kératiniques, comprenant dans un milieu approprié pour la teinture a) au moins une base d'oxydation et/ou au moins un colorant direct et b) au moins un acide polycarboxylique particulier ou un de ses sels. Elle concerne de plus une composition prête à l'emploi la comprenant ainsi qu'un procédé de mise en oeuvre et un dispositif.

Pour colorer les fibres kératiniques, plus particulièrement les fibres kératiniques humaines, telles que les cheveux notamment, il est connu d'utiliser des compositions tinctoriales contenant des précurseurs de colorant d'oxydation, appelés généralement bases d'oxydation, des colorants directs ou encore leurs associations. Les bases d'oxydation sont des précurseurs incolores ou faiblement colorés qui, associés à des produits oxydants, peuvent donner naissance par un processus de condensation oxydative à des composés colorés et colorants. On peut faire varier les nuances obtenues avec ces bases d'oxydation en les associant à des coupleurs ou modificateurs de coloration. Les colorants directs sont, quant à eux, des molécules colorées et colorantes ayant une affinité pour les fibres. Lorsque les colorants directs sont appliqués en présence d'un agent oxydant, on parle de coloration éclaircissante.

Habituellement, les compositions tinctoriales comprennent au moins un agent séquestrant choisi parmi l'acide éthylènediamine tétraacétique (EDTA) et ses dérivés comme l'acide diéthylènetriamine pentaacétique (DPTA). Le rôle de ces agents est de complexer les cations métalliques susceptibles de se trouver à l'état de traces dans les compositions, ainsi que ceux pouvant être présents sur les cheveux et provenant de l'air ambiant, de l'eau avec laquelle ces derniers ont été lavés ou encore des shampoings ou autres produits capillaires avec lesquels ils ont été traités. Il est, en effet, très important de neutraliser ces cations métalliques, dans la mesure où ils sont susceptibles de catalyser les réactions d'oxydation au niveau des fibres capillaires et ce, de façon non contrôlée, ce qui peut se traduire par des effets indésirables sévères tels qu'une cassure des cheveux ou une brûlure du cuir chevelu.

Les compositions comprenant de tels agents séquestrants donnent de bonnes propriétés tinctoriales mais qui peuvent être encore améliorées.

Ainsi, la présente invention a pour objet une composition destinée à la coloration des fibres kératiniques, comprenant dans un milieu approprié pour la teinture :
a) au moins une base d'oxydation et/ou au moins un colorant direct ;
b) au moins un composé de formule générale (I) suivante :

   R-N-(CH(R')CO₂X)₂ (I)

   dans laquelle :
   - R représente un atome d'hydrogène ou un groupe CH(CO₂X)-(CH₂)₂CO₂X, CH₂CH₂OH, CH(CH₃)-CO₂X ou (CH₂)₂-N(COR")-CH₂-CO₂X;
   - R" représente un groupe alkyle linéaire ou ramifié, comportant de 1 à 30 atomes de carbone ou cyclique comportant de 3 à 30 atomes de carbone ;
   - R' représente un groupe CH₂CO₂X si R représente un atome d'hydrogène ou R' représente un atome d'hydrogène si R est différent d'un atome d'hydrogène ; et
   - X représente un atome d'hydrogène ou un cation monovalent ou divalent choisi parmi ceux issus des métaux alcalins, alcalino-terreux, des métaux de transition, des amines organiques ou d'ions ammonium.

L'invention a de même pour objet une composition prête à l'emploi comprenant la composition précitée et une composition oxydante comprenant, dans un milieu approprié pour la teinture, au moins un agent oxydant.

Un autre objet de l'invention consiste en un procédé de coloration de fibres kératiniques, comprenant successivement les étapes suivantes :
a) on applique sur les fibres kératiniques une composition comprenant, dans un milieu approprié pour la teinture, au moins une base d'oxydation et/ou au moins un colorant direct et au moins un composé de formule générale (I) précitée, et éventuellement une composition oxydante, celles-ci étant mélangées extemporanément avant l'emploi ou appliquées successivement ;
b) on laisse reposer pendant un temps suffisant pour obtenir la coloration recherchée
c) on rince les fibres kératiniques pour en éliminer la composition selon l'invention et éventuellement la composition oxydante ;
d) on lave éventuellement les fibres kératiniques, on les rince ;
e) on les sèche éventuellement.

Enfin, l'invention a pour objet un dispositif pour la coloration de fibres kératiniques, comprenant au moins deux compartiments, l'un d'eux comprenant une composition selon l'invention, l'autre comprenant une composition oxydante comprenant dans un milieu approprié pour la teinture, au moins un agent oxydant.

On a en effet constaté que la présence de composé(s) correspondant(s) à ceux de formule (I) et utilisés comme séquestrants dans des compositions tinctoriales, permettait d'améliorer la montée du colorant dans le cheveu et/ou de diminuer les écarts de coloration (diminution de la sélectivité) entre les cheveux ou portions de cheveux différemment sensibilisés.
Rappelons qu'un cheveu est sensibilisé lorsqu'il a subi des traitements de coloration, décoloration ou mise en forme permanente.

Par ailleurs, les nuances obtenues présentent une bonne ténacité vis-à-vis des agressions extérieures (shampooing, lumière, permanentes, etc.).

En outre, le composé de formule (I) utilisé en tant qu'agent complexant dans la composition de coloration selon l'invention présente l'avantage d'être d'une innocuité totale pour la peau, notamment d'être dénué de tout caractère allergène.
Par ailleurs, il possède une bonne biodégradabilité, et son coût de production ou d'achat autorise son utilisation dans des compositions destinées à être vendues, non seulement à des professionnels, mais également dans la distribution grand public.

Mais d'autres caractéristiques et avantages de la présente invention apparaîtront plus clairement à la lecture de la description et des exemples qui vont suivre.

Comme cela a été indiqué précédemment, la composition selon l'invention comprend, dans un milieu approprié pour la teinture, au moins une base d'oxydation et/ou au moins un colorant direct.

### Bases d'oxydation/coupleurs

La base d'oxydation peut être choisie parmi les bases d'oxydation classiquement utilisées pour les colorations d'oxydation, comme par exemple les paraphénylène-diamines, les bis-phénylalkylènediamines, les para-aminophénols, les orthoamino-phénols et les bases hétérocycliques et leurs sels d'addition avec un acide ou avec un agent alcalin, seules ou en mélanges.

Parmi les paraphénylènediamines, on peut plus particulièrement citer à titre d'exemple, la paraphénylènediamine, la paratoluylènediamine, la 2-chloro para-phénylènediamine, la 2,3-diméthyl paraphénylènediamine, la 2,6-diméthyl para-phénylènediamine, la 2,6-diéthyl paraphénylènediamine, la 2,5-diméthyl para-phénylènediamine, la N,N-diméthyl paraphénylènediamine, la N,N-diéthyl para-phénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para phénylènediamine, la 2-fluoro paraphénylènediamine, la 2-isopropyl para phénylènediamine, la N-(β-hydroxy- propyl) paraphénylènediamine, la 2-hydroxyméthyl paraphénylènediamine, la N,N-diméthyl 3-méthyl paraphénylènediamine, la N,N-(éthyl, β-hydroxyéthyl) para-phénylène diamine, la N-(β,γ-dihydroxypropyl) para phénylènediamine, la N-(4'-aminophényl) paraphénylènediamine, la N-phényl para phénylènediamine, la 2-β-hydroxyéthyloxy paraphénylènediamine, la 2-β-acétylaminoéthyloxy paraphénylènediamine, la N-(β-méthoxyéthyl) paraphénylènediamine et la 4'aminophényl 1-(3-hydroxy) pyrrolidine, et leurs sels d'addition avec un acide ou avec un agent alcalin.

Parmi les bis-phénylalkylènediamines, on peut plus particulièrement citer à titre d'exemple, le N,N'-bis-((3-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-amino phényl) tétraméthylènediamine, la N,N'-bis-(β-hydroxyéthyl) N,N'-bis-(4-aminophényl) tétraméthylènediamine, la N,N'-bis-(4-méthylamino phényl) tétraméthylènediamine, la N,N'-bis-(éthyl) N,N'-bis-(4'-amino, 3'-méthyl phényl) éthylènediamine, le 1,8-bis-(2,5-diaminophénoxy)-3,5-dioxaoctane, et leurs sels d'addition avec un acide ou avec un agent alcalin.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxy méthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide ou avec un agent alcalin.

Parmi les ortho-aminophénols, on peut citer par exemple, le 2-amino phénol, le 2-amino 5-méthyl phénol, le 2-amino 6-méthyl phénol, le 5-acétamido 2-amino phénol, et leurs sels d'addition avec un acide ou avec un agent alcalin.

Parmi les bases hétérocycliques, on peut notamment citer les dérivés pyridiniques, les dérivés pyrimidiniques et les dérivés pyrazoliques et leurs sels d'addition avec un acide ou avec un agent alcalin.

Habituellement, lorsque la composition comprend une ou plusieurs bases d'oxydation, la teneur en ces composés varie entre 0,0005 et 12 % en poids par rapport au poids total de la composition, et de préférence de 0,005 et 6 % en poids par rapport à la même référence.

Si l'on souhaite faire varier les nuances obtenues avec ces bases d'oxydation, il est souvent avantageux de les associer à au moins un coupleur.

Les coupleurs peuvent notamment être choisis parmi les métaphénylènediamines, les méta-aminophénols, les métadiphénols et les coupleurs hétérocycliques et leurs sels d'addition avec un acide ou avec un agent alcalin.

Conviennent plus particulièrement les coupleurs choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1 ,5-b]-1 ,2,4-triazole, le 2,6-diméthyl [3,2-c]-1,2,4-triazole, le 6-méthyl pyrazolo [1,5-a]-benzimidazole, et leurs sels d'addition avec un acide ou avec un agent alcalin.

Lorsqu'ils sont présents, le ou les coupleurs représentent par exemple de 0,0001 à 10 % en poids par rapport au poids total de la composition, et de préférence de 0,005 à 5 % en poids par rapport à la même référence.

D'une manière générale, les sels d'addition avec un acide utilisables dans le cadre des compositions de l'invention sont notamment choisis parmi les chlorhydrates, les bromhydrates, les sulfates, les citrates, les succinates, les tartrates, les tosylates, les benzènesulfonates, les lactates et les acétates.
Les sels d'addition avec un agent alcalin utilisables dans le cadre de l'invention sont notamment choisis parmi les sels d'addition avec les métaux alcalins ou alcalino-terreux, avec l'ammoniaque, avec les amines organiques dont les alcanolamines et les composés suivants de formule : 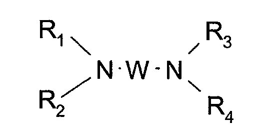 dans laquelle W est un reste propylène éventuellement substitué par un groupement hydroxyle ou un radical alkyle en C₁-C₆ ; R₁, R₂, R₃ et R₄, identiques ou différents, représentent un atome d'hydrogène, un radical alkyle en C₁-C₆ éventuellement porteur d'au moins un radical hydroxyle.

### Colorants directs

La composition selon l'invention peut comprendre, à la place ou en association avec la ou les bases d'oxydation éventuellement associée(s) à un ou plusieurs coupleurs, au moins un colorant direct.

Les colorants directs sont plus particulièrement des composés absorbant les radiations lumineuses dans le domaine visible (400-750 nm).
Ils peuvent être de nature non ionique, anionique ou cationique.

Généralement, ces colorants directs sont choisis parmi les colorants benzéniques nitrés, les colorants azoïques, anthraquinoniques, naphtoquinoniques, benzoquinoniques, phénotiaziniques indigoïdes, xanthéniques, phénanthridiniques, phtalocyanines, ceux dérivés du triarylméthane, seuls ou en mélanges.

Parmi les colorants benzéniques nitrés, on peut citer les composés rouges ou orangés suivants : le 1-hydroxy-3-nitro-4-N-(γ-hydroxypropyl)amino benzène, le N-(β-hydroxy éthyl)amino-3-nitro-4-amino benzène, le 1-amino-3-méthyl-4-N-(β-hydroxyéthyl)amino-6-nitro benzène, le 1-hydroxy-3-nitro-4-N-(β-hydroxyéthyl)-amino benzène, le 1,4-diamino-2-nitrobenzène, le 1-amino-2-nitro-4-méthylamino benzène, la N-(β-hydroxyéthyl)-2-nitro-paraphénylènediamine, le 1-amino-2-nitro-4-(β-hydroxy éthyl)amino-5-chloro benzène, la 2-nitro-4-amino-diphénylamine, le 1-amino-3-nitro-6-hydroxybenzène, le 1-(β-amino éthyl)amino-2-nitro-4-([β-hydroxy éthyloxy) benzène, le 1-(β, γ-dihydroxypropyl)oxy-3-nitro-4-(β-hydroxyéthyl)amino benzène, le 1-hydroxy-3-nitro-4-aminobenzène, le 1-hydroxy-2-amino-4,6-dinitrobenzène, le 1-méthoxy-3-nitro-4-(β-hydroxyéthyl)amino benzène, la 2-nitro-4'-hydroxydiphénylamine, le 1-amino-2-nitro-4-hydroxy-5-méthylbenzène, seuls ou en mélanges.

En ce qui concerne les colorants directs benzéniques nitrés, on peut mettre en oeuvre des colorants de ce type jaunes et jaune-verts, comme par exemple le 1-β-hydroxyéthyloxy-3-méthylamino-4-nitrobenzène, le 1-méthylamino-2-nitro-5-([β,γ-dihydroxypropyl)oxy benzène, le 1-(β-hydroxyéthyl)amino-2-méthoxy-4-nitrobenzène, le 1-(β-amino éthyl)amino-2-nitro-5-méthoxy-benzène, le 1,3-di(β-hydroxyéthyl)amino-4-nitro-6-chloro benzène, le 1-amino-2-nitro-6-méthyl-benzène, le 1-(β-hydroxyéthyl)amino-2-hydroxy-4-nitrobenzène, la N-(β-hydroxyéthyl)-2-nitro-4-trifluorométhylaniline, l'acide 4-(β-hydroxy éthyl)amino-3-nitro-benzènesulfonique, l'acide 4-éthylamino-3-nitro-benzoïque, le 4-(β-hydroxyéthyl)amino-3-nitro-chlorobenzène, le 4-(β-hydroxyéthyl)amino-3-nitro-méthyl benzène, le 4-(β,γ-dihydroxypropyl)amino-3-nitro-trifluorométhylbenzène, le 1-(β-uréido éthyl)amino-4-nitrobenzène, le 1,3-diamino-4-nitrobenzène, le 1-hydroxy-2-amino-5-nitrobenzène, le 1-amino-2-[tris(hydroxyméthyl)méthyl]amino-5-nitro-benzène, le 1-(β-hydroxyéthyl)amino-2-nitrobenzène, le 4-((3-hydroxyéthyl)amino-3-nitrobenzamide.

Il est de même envisageable d'utiliser des colorants benzéniques nitrés bleus ou violets, comme entre autres le 1-(β-hydroxyéthyl)amino-4-N,N-bis-(β-hydroxyéthyl)amino 2-nitrobenzène, le 1-(γ-hydroxypropyl)amino 4-N,N-bis-(β-hydroxyéthyl)amino 2-nitrobenzène, le 1-(β-hydroxyéthyl)amino 4-(N-méthyl, N-β-hydroxyéthyl)amino 2-nitrobenzène, le 1-(β-hydroxyéthyl)amino 4-(N-éthyl, N-β-hydroxyéthyl)amino 2-nitrobenzène, le 1-(β,γ-dihydroxypropyl)amino 4-(N-éthyl, N-β-hydroxyéthyl)amino 2-nitrobenzène, les 2-nitroparaphénylènediamines de formule suivante : 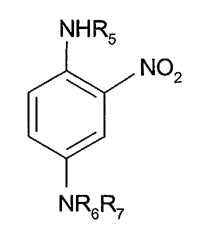 dans laquelle :
- R₆ représente un radical alkyle en C₁-C₄, un radical β-hydroxyéthyle ou β-hydroxypropyle ou γ-hydroxypropyle ;
- R₅ et R₇, identiques ou différents, représentent un radical β-hydroxyéthyle, β-hydroxypropyle, γ-hydroxypropyle, ou β,γ-dihydroxypropyle, l'un au moins des radicaux R₆, R₇ ou R₅ représentant un radical γ-hydroxypropyle et R₆ et R₇ ne pouvant désigner simultanément un radical β-hydroxyéthyle lorsque R₆ est un radical γ-hydroxypropyle, telles que celles décrits dans le brevet français FR 2 692 572.

Il est rappelé que les colorants azoïques sont des composés comportant dans leur structure au moins un enchaînement -N=N- non inclus dans un cycle ; les colorants méthiniques sont des composés comportant dans leur structure au moins un enchaînement -C=C- non inclus dans un cycle ; les colorants azométhinique sont des composés comportant dans leur structure au moins un enchaînement -C=N- non inclus dans un cycle.

Les colorants dérivés de triarylméthane comportent dans leur structure au moins un enchaînement suivant : 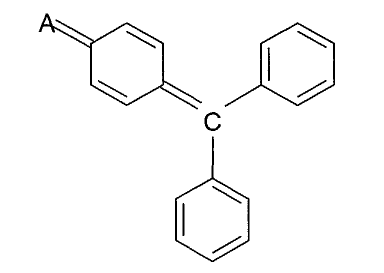 A désignant un atome d'oxygène ou d'azote
Les colorants xanthéniques comportent dans leur structure au moins un enchaînement de formule :

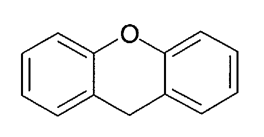

Les colorants phénanthridiniques comportent dans leur structure au moins un enchaînement de formule

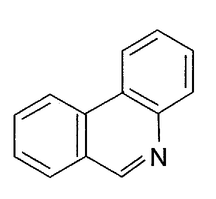

Les colorants phtalocyanines comportent dans leur structure au moins un enchaînement de formule :

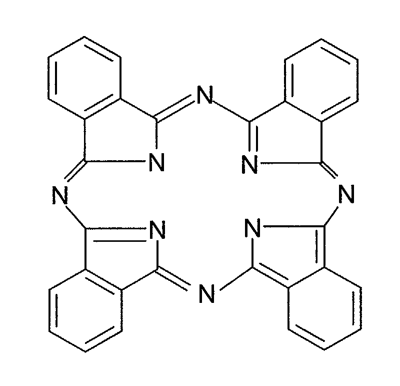

Les colorants phénotiaziniques comportent dans leur structure au moins un enchaînement suivant :

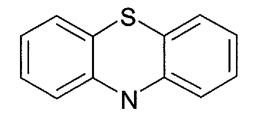

Les colorants directs peuvent de plus être choisis parmi les colorants basiques comme ceux listés dans le Color Index, 3ème édition, notamment sous les dénominations "Basic Brown 16", "Basic Brown 17", "Basic Yellow 57", "Basic Red 76", "Basic Violet 10", "Basic Blue 26" et "Basic Blue 99" ; ou parmi les colorants directs acides, listés le Color Index, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore les colorants directs cationiques tels que ceux décrits dans les demandes de brevet WO 95/01772, WO 95/15144 et EP 714954 et dont le contenu fait partie intégrante de la présente invention.

Lorsqu'ils sont présents, le ou les colorants directs représentent de préférence de 0,0005 à 12 % en poids environ du poids total de la composition, et encore plus préférentiellement de 0,005 à 6 % en poids environ de ce poids.

### Composé complexant

La composition comprend de plus, au moins un composé de formule (I) suivante :

R-N-(CH(R')CO₂X)₂ (I)

dans laquelle :
- R représente un atome d'hydrogène ou un groupe CH(CO₂X)-(CH₂)₂CO₂X, CH₂-CH₂-OH, CH(CH₃)-CO₂X ou (CH₂)₂-N(COR")-CH₂-CO₂X;
- R" représente un groupe alkyle linéaire ou ramifié, comportant de 1 à 30 atomes de carbone ou cyclique comportant de 3 à 30 atomes de carbone ;
- R' représente un groupe CH₂CO₂X si R représente un atome d'hydrogène ou R' représente un atome d'hydrogène si R est différent d'un atome d'hydrogène ; et
- X représente un atome d'hydrogène ou un cation monovalent ou divalent choisi parmi ceux issus des métaux alcalins, alcalino-terreux, des métaux de transition, des amines organiques ou d'ions ammonium.

Ainsi, les agents complexants utilisés dans le cadre de l'invention, correspondent à des composés acides polycarboxyliques et aux carboxylates correspondants.

Plus précisément, les agents complexants correspondent à :
- des composés comprenant quatre fonctions acides carboxyliques ou carboxylates, lorsque R représente un atome d'hydrogène et R' représente un groupe -CH₂-CO₂X, ou lorsque R représente le groupe -CH(CO₂X)-(CH₂)₂-CO₂X et R' représente un atome d'hydrogène ;
- des composés comprenant trois fonctions acides carboxyliques ou carboxylates, lorsque R représente le groupe -CH(CH₃)-CO₂X et R' représente un atome d'hydrogène, ou lorsque R représente un groupe -(CH₂)₂-N(COR")-CH₂-CO₂X et R' représente un atome d'hydrogène ; et à
- des composés comprenant deux fonctions acides carboxyliques ou carboxylates, lorsque R représente le groupe -CH₂-CH₂-OH et R' représente un atome d'hydrogène.

A titre d'exemples de cations de métaux alcalins, on peut notamment citer le sodium (Na⁺) et le potassium (K⁺), et à titre d'exemples de cations de métaux alcalino-terreux, on peut notamment citer le calcium (Ca²⁺) et le magnésium (Mg²⁺).

Au sens de la présente invention, on entend par "métal de transition", un métal comportant une sous-couche d incomplète, plus particulièrement à l'état d'oxydation II, tel que le cobalt (Co²⁺), le fer (Fe²⁺), le manganèse (Mn²⁺), le zinc (Zn²⁺) et le cuivre (Cu²⁺).

En ce qui concerne les sels d'amines organiques, on peut citer les sels d'amine primaire, secondaire ou tertiaire, ou encore d'alcanolamine.
Lesdites amines présentent un ou plusieurs radicaux, identiques ou non, de type alkyle, linéaire ou ramifié en C₁ à C₂₀, comprenant éventuellement un hétéroatome comme l'oxygène.

Pour ce qui a trait aux sels d'ammonium quaternaires , ces derniers comprenant trois radicaux, identiques ou non, choisis parmi l'hydrogène, un radical alkyle, linéaire ou ramifié en C₁ à C₂₀, comprenant éventuellement un hétéroatome comme l'oxygène.

Le ou les composés de formule (I) sont, de préférence, choisis dans le groupe constitué par l'acide méthylglycine diacétique, l'acide 2-hydroxy-éthyliminodiacétique, l'acide N-lauroyl-N,N',N'-triacétique éthylènediamine, l'acide iminodisuccinique, l'acide N,N-dicarboxyméthyl L-glutamique, leurs sels de métaux alcalins, leurs sels de métaux alcalino-terreux, leurs sels de métaux de transition, et leurs mélanges.

Conformément à un mode de réalisation particulièrement avantageux de l'invention, le ou les composés de formule (I) sont choisis dans le groupe constitué par l'acide méthylglycine diacétique, l'acide N-lauroyl-N,N',N'-triacétique éthylènediamine, l'acide N,N-dicarboxyméthyl L-glutamique, leurs sels de métaux alcalins, leurs sels de métaux alcalino-terreux, leurs sels de métaux de transition, et leurs mélanges.

L'acide méthylglycine diacétique, l'acide 2-hydroxyéthyliminodiacétique, l'acide N-lauroyl-N',N'-triacétique éthylènediamine, l'acide iminodisuccinique, l'acide N,N-dicarboxyméthyl L-glutamique et leurs sels sont respectivement représentés par les formules (II), (III), (IV), (V), (VI) suivantes :

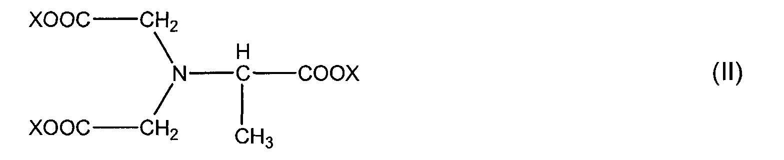

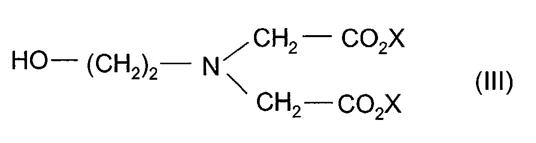

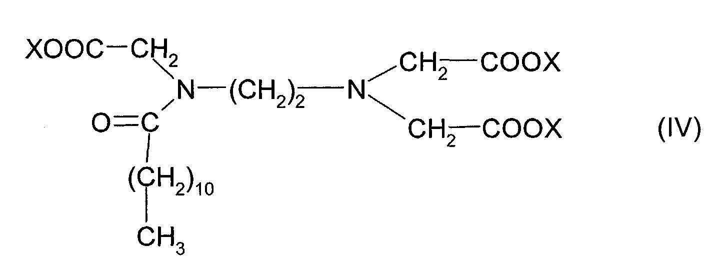

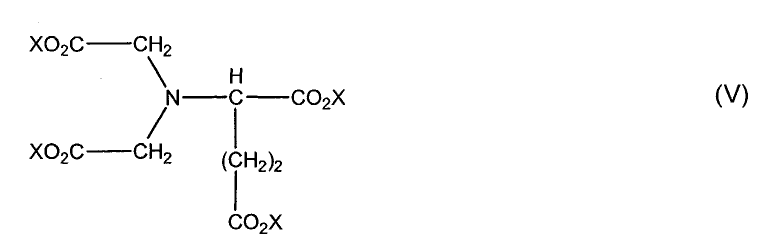

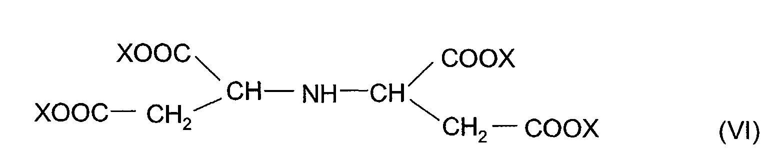

Formules dans lesquelles X est tel que défini précédemment, X correspondant, de préférence, à H ou Na.

Ces composés sont notamment disponibles auprès des sociétés BASF, DOW CHEMICAL, HAMPSHIRE, BAYER et SHOWA DENKO.

Plus particulièrement, on préfère l'acide méthylglycine-diacétique, ses sels de sodium et leurs mélanges.

La teneur en composé de formule (I) est plus particulièrement comprise entre 0,001 et 10 % en poids par rapport au poids total de la composition, de préférence entre 0,001 et 5 % en poids par rapport à la même référence. Les pourcentages sont exprimés par rapport à la forme acide du ou des composés de formule (I).

### Milieu

Le milieu de la composition, approprié pour la teinture, est plus particulièrement un milieu aqueux, c'est-à-dire comprenant de l'eau et éventuellement un ou plusieurs solvants organiques acceptables sur le plan cosmétique.

A titre d'exemples, lesdits solvants peuvent être en particulier choisis parmi les monoalcools ou les diols, linéaire ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C₁-C₄, comme par exemple, le monoéthyléther ou le monobutyléther du diéthylèneglycol, seuls ou en mélange.

Le ou les solvants organiques, lorsqu'ils sont présents dans la composition selon l'invention, représentent généralement de 0,5 à 20% en poids par rapport au poids total de cette composition et de préférence de 2 à 10% en poids par rapport à la même référence.

La composition peut de plus comprendre les additifs classiques dans le domaine.

### Polymères conditionneurs

La composition selon l'invention peut notamment comprendre au moins un polymère conditionneurs cationiques ou amphotères, ou leurs mélanges. Rappelons que, au sens de la présente invention, l'expression "polymère conditionneur cationique" désigne tout polymère contenant des groupements cationiques et/ou des groupements ionisables en groupements cationiques et qui permet d'améliorer les propriétés cosmétiques des fibres kératiniques, en particulier le démêlage, la douceur, la brillance, le volume.

Les polymères cationiques ou amphotères convenables sont de manière avantageuse, choisis parmi ceux déjà connus en soi comme améliorant les propriétés cosmétiques des cheveux, à savoir notamment ceux décrits dans les brevets et demandes de brevet EP 337 354, FR 2 270 846, FR 2 383 660, FR 2 598 611, FR 2 470 596, FR 2 519 863, FR 2 788 974, FR 2 788 976 pour avoir une liste de ces composés.

Cependant, à titre d'exemples plus précis de polymères cationiques, on peut notamment citer les polymères cationiques comprenant au moins des groupements amine primaire, secondaire, tertiaire et/ou quaternaire pouvant, soit faire partie de la chaîne principale polymère, soit être portés par un substituant latéral directement relié à celle-ci.

A titre d'exemples plus précis de ces polymères, on peut citer :
(1) les copolymères d'acrylamide et de diméthyl-amino-éthyl méthacrylate quaternisé au sulfate de diméthyle ou avec un halogénure de diméthyle (Hercofloc de Hercules) ; les copolymères d'acrylamide et de chlorure de méthacryloyloxy-éthyl-triméthylammonium (Bina Quat P 100 de Ciba Geigy) ; le copolymère d'acrylamide et de méthosulfate de méthacryloyloxy-éthyl-triméthylammonium (Reten de Hercules) ; les copolymères vinylpyrrolidone / acrylate ou méthacrylate de dialkylaminoalkyle quaternisés ou non (gamme Gafquat d'ISP ; Copolymer 845, 958 et 937 de Gaf Corporation (ISP) ; les terpolymères méthacrylate de diméthyl amino éthyle/ vinylcaprolactame/ vinylpyrrolidone (Gaffix VC 713 d'ISP) ; les copolymères vinylpyrrolidone / méthacrylamidopropyl dimethylamine (Styleze CC 10 d'ISP) ; les copolymères vinylpyrrolidone / méthacrylamide de diméthyl-amino-propyle quaternisés (Gafquat HS 100 d'ISP).
(2) Les dérivés d'éthers de cellulose comportant des groupements ammonium quaternaire tels que décrits dans FR 1 492 597. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans US 4 131 576, tels que les hydroxyalkylcelluloses, comme les hydroxyméthyl-, hydroxyéthyl- ou hydroxypropylcelluloses greffées notamment avec un sel de méthacryloyléthyl triméthylammonium, de méthacrylamidopropyl-triméthylammonium, de diméthyl-diallylammonium.
(4) Les polysaccharides cationiques décrits plus particulièrement dans les brevets US 3 589 578, US 4 031 307, tels que les gommes de guar contenant des groupements cationiques trialkylammonium. On utilise par exemple des gommes de guar modifiées par un sel, comme le chlorure notamment, de 2,3-époxypropyl triméthylammonium.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans FR 2 162 025, FR 2 280 361.
(6) Les polyaminoamides solubles dans l'eau préparés en particulier par polycondensation d'un composé acide avec une polyamine, éventuellement réticulés, éventuellement alcoylés ou s'ils comportent une ou plusieurs fonctions amines tertiaires, quaternisées. Ces polymères sont notamment décrits dans FR 2 252 840 et FR 2 368 508 .
(7) Les dérivés de polyaminoamides résultant de la condensation de polyalkylènes polyamines avec des acides polycarboxyliques suivie d'une alcoylation par des agents bifonctionnels. On peut citer par exemple les polymères acide adipique-dialkylamino hydroxyalkyldialkylène triamine dans lesquels le radical alkyle est en C1-C4. De tels polymères sont notamment décrits dans FR 1 583 363.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés en C3-C8, puis avec l'épichlorhydrine. De tels polymères sont notamment décrits dans US 3 227 615, US 2 961 347.
(9) Les cyclopolymères d'alkyl diallyl amine ou de dialkyl diallyl ammonium, sous forme d'homopolymère ou de copolymère, tels que décrits dans FR 2 080 759 et dans son certificat d'addition n°2 190 406.
(10) Les polymères de diammonium quaternaire tels que décrits dans FR 2 320 330, FR 2 270 846, FR 2 316 271, FR 2 336 434, FR 2 413 907, US 2 273 780, US 2 375 853, US 2 388 614, US 2 454 547, US 3 206 462, US 2 261 002, US 2 271 378, US 3 874 870, US 4 001 432, US 3 929 990, US 3 966 904, US 4 005 193, US 4 025 617, US 4 025 627, US 4 025 653, US 4 026 945, US 4 027 020.
   On peut utiliser plus particulièrement les polymères qui sont constitués de motifs récurrents répondant à la formule suivante: 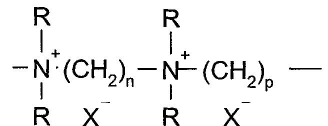 dans laquelle les radicaux R, identiques ou non, désignent un radical alkyle ou hydroxyalkyle en C1-C4, n et p sont des nombres entiers variant de 2 à 20 et, X- est un anion dérivé d'un acide minéral ou organique.
(11) Les polymères de poly(ammonium quaternaire) constitués de motifs récurrents de formule : 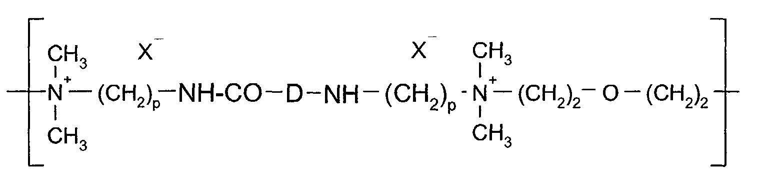 dans laquelle p désigne un nombre entier variant de 1 à 6 environ, D peut être nul ou peut représenter un groupement -(CH₂)ᵣ -CO- dans lequel r désigne un nombre égal à 4 ou à 7, X- est un anion. De tels polymères peuvent être préparés selon les procédés décrits dans US 4 157 388, US 4 702 906, US 4 719 282, EP 122 324.
(12) Les polymères quaternaires de vinylpyrrolidone et de vinylimidazole.
(13) Les polyamines du type Polyethyleneglycol (15) Tallow Polyamine(dénomination du dictionnaire CTFA).
(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale (Salcare® SC 92 de Ciba). On peut également utiliser un homopolymère réticulé du chlorure de méthacryloyloxyéthyl triméthylammonium contenant environ 50 % en poids de l'homopolymère dans de l'huile minérale ou dans un ester liquide (Salcare® SC 95, SC 96 de Ciba).

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

En ce qui concerne les polymères amphotères, on peut utiliser des polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a réagi avec une polyamine comportant un ou plusieurs groupements amine primaire ou secondaire.

Les polymères amphotères répondant à la définition donnée ci-dessus plus particulièrement préférés, sont choisis parmi les polymères suivants :
**(1)** Les polymères résultant de la copolymérisation d'un monomère dérivé d'un composé vinylique portant un groupement carboxylique tel que plus particulièrement l'acide (méth)acrylique, l'acide maléique, l'acide alpha-chloracrylique, ou encore un sel de dialkyldiallylammonium tel que le chlorure de diméthyldiallylammonium, et d'un monomère basique dérivé d'un composé vinylique substitué contenant au moins un atome basique tel que plus particulièrement les dialkyl-amino-alkyl-méthacrylate et acrylate, les dialkylamino-alkyl-méthacrylamide et acrylamide, comme décrits dans US 3 836 537. On peut citer le copolymère acrylate de sodium / chlorure d'acrylamidopropyl trimethyl ammonium (Polyquart KE 3033 de Cognis), le copolymère acide acrylique/ chlorure de diméthyldiallylammonium (Merquat 280, 295, Plus 3330, de Nalco).
**(2)** Les polymères comportant des motifs dérivant a) d'au moins un monomère choisi parmi les (méth)acrylamides N-substitués par un radical alkyle, notamment en C₂-C₁₂ (par exemple éthyl, tertiobutyl, tertiooctyl, octyl, décyl, le dodécyl), b) d'au moins un monomère acide contenant un ou plusieurs groupements carboxyliques réactifs (par exemple acides (méth)acrylique, crotonique, itaconique, et les monoesters des acides ou anhydrides maléique, fumarique), et c) d'au moins un monomère basique tel que des esters à substituant amine primaire, secondaire, tertiaire et quaternaire des acides (méth)acrylique, fumarique, maléique, et le produit de quaternisation du méthacrylate de diméthyl-amino-éthyle avec le sulfate de diméthyle ou diéthyle (par exemple les méthacrylates d'aminoéthyle, de butylaminoéthyle, de N,N'-diméthyl-aminoéthyle, de N-tertio-butyl-aminoéthyle.)
   On utilise particulièrement les copolymères octylacrylamide / acrylate / butylaminoéthylméthacrylate (Amphomer ou Lovocryl 47 par la société National Starch).
**(3)** Les polyaminoamides réticulés et partiellement ou totalement alcoylés, dérivant de polyaminoamides de formule générale -[CO-R-CO-Z]- dans laquelle R est un radical divalent dérivé d'un acide dicarboxylique saturé ou non (par exemple les acides adipique, triméthyl-2,2,4-adipique et triméthyl-2,4,4-adipique, téréphtalique, itaconique), d'un acide monocarboxylique insaturé (comme l'acide (méth)acrylique), d'un ester d'alcool en C₁-C₆ des acides précités ou d'un radical dérivant de l'addition de l'un de ces acides avec une amine bis-primaire ou -secondaire, et Z désigne un radical d'une polyalkylène-polyamine bis-primaire, mono ou bis-secondaire. De préférence, Z représente entre 60 et 100 moles %, le radical -NH-[(CH₂)x-NH]p- avec x=2 et p=2 ou 3, ou x=3 et p=2 ; ce radical dérivant de la diéthylène triamine, de la triéthylène tétraamine ou de la dipropylène triamine; entre 0 et 40 moles % le radical ci-dessus, dans lequel x=2 et p=1 et qui dérive de l'éthylènediamine, ou le radical dérivant de la pipérazine -N[CH₂CH₂]₂N- ; entre 0 et 20 moles %, le radical -NH-(CH2)6-NH- dérivant de l'hexaméthylènediamine. L'agent réticulant de ces polymères est un agent bifonctionnel choisi parmi les épihalohydrines, les diépoxydes, les dianhydrides, les dérivés bis insaturés, et alcoylés par action d'acide acrylique, d'acide chloracétique ou d'une alcane sultone (comme la propane ou la butane sultone) ou de leurs sels de métaux alcalins.
**(4)** Les polymères comportant au moins des motifs zwittérioniques, comme par exemple le copolymère de méthacrylate de butyle / méthacrylate de diméthylcarboxy-méthylammonio-éthyle (Diaformer Z301 de Sandoz).
**(5)** Les polymères dérivés du chitosane comportant des motifs monomères répondant aux formules (A), (B), (C) suivantes : 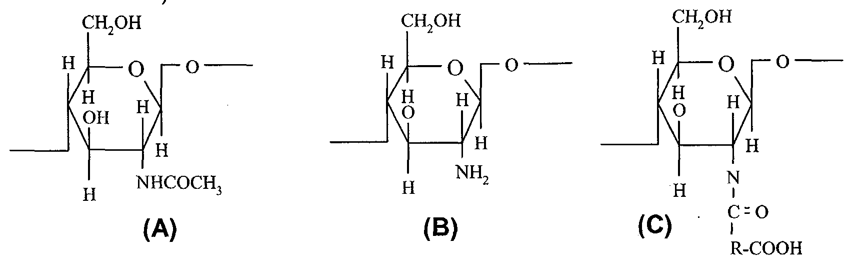 avec (A) représentant de 0 à 30%, (B) de 5 à 50% et (C) de 30 à 90% dans lequel R représente un radical de formule : 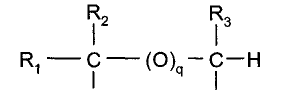 dans laquelle q désigne zéro ou 1 ; et si q=0, Ri, identiques ou non, représentent un hydrogène, un groupement méthyle, hydroxyle, acétoxy, amino, mono- ou di-alkylamine éventuellement interrompus par un ou plusieurs atomes d'azote et/ou éventuellement substitués par un ou plusieurs groupes amine, hydroxyle, carboxyle, alkylthio éventuellement porteur d'un groupe amino, sulfonique ; ou si q=1, Ri, identiques ou non, représentent un hydrogène, ainsi que les sels formés par ces composés avec des bases ou des acides.
**(6)** Les polymères dérivés de la N-carboxyalkylation du chitosane comme le N-carboxyméthyl chitosane ou le N-carboxybutyl chitosane (Evalsan de Jan Dekker).
**(7)** Les polymères tels que décrits dans FR 1 400 366 : 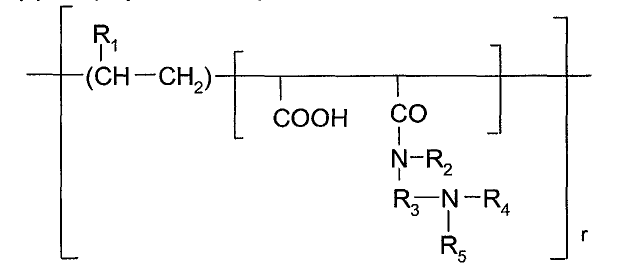 dans laquelle R₁ est un hydrogène, CH₃O-, CH₃CH₂O-, phényle, R₂, R₅, identiques ou non, représentent un hydrogène, un radical alkyle (méthyle, éthyle), R₄ représente un radical alkyle (méthyle, éthyle) ou un radical de formule -R₃-N(R₅)₂, R₃ représentant -(CH₂)₂-, -(CH2)3-, -CH₂-CH(CH₃)-, ainsi que les homologues supérieurs de ces radicaux et contenant jusqu'à 6 atomes de carbone, r est tel que le poids moléculaire est compris entre 500 et 6000000 et de préférence entre 1000 et 1000000.
**(8)** Les polymères amphotères du type -D-X-D-X- choisis parmi:
   a) Les polymères obtenus par action de l'acide chloracétique ou le chloracétate de sodium sur les composés comportant au moins un motif de formule -D-X-D-X-D- où D désigne un radical -N[CH₂CH₂]₂N- (pipérazinyle) et X désigne le symbole E ou E', E ou E' identiques ou différents désignent un radical bivalent qui est un radical alkylène à chaîne droite ou ramifiée comportant jusqu'à 7 atomes de carbone dans la chaîne principale substituée ou non par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule -D-X-D-X- où D désigne un radical -N[CH₂CH₂]₂N-(pipérazinyle) et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
**(9)** Les copolymères alkyl(C₁-C₅)-vinyléther / anhydride maléique modifié partiellement par semi-amidification avec une N,N-dialkylaminoalkylamine telle que la N,N-diméthylamino-propylamine ou par semi-estérification avec une N,N-dialcanolamine. Ces copolymères peuvent également comporter d'autres comonomères vinyliques tels que le vinylcaprolactame.

Parmi tous les polymères cationiques ou amphotères utilisables selon la présente invention, on préfère notamment :
(i) parmi les cationiques :
   - l'homopolymère de chlorure de diméthyldiallylammonium (Merquat 100 de Nalco);
   - les copolymères de chlorure de diméthyldiallylammonium et d'acrylamide (Merquat 2200 de Nalco);
   - les polymères de type poly(ammonium quaternaire) préparés et décrits dans FR 2 270 846. constitués de motifs récurrents de formules (W) et (U) suivantes : 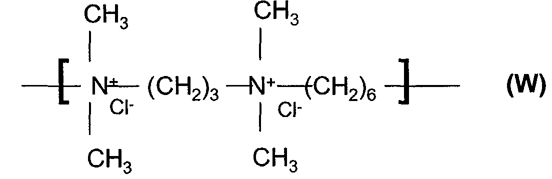 et notamment ceux dont le poids moléculaire, déterminé par chromatographie par perméation de gel, est compris entre 9500 et 9900: 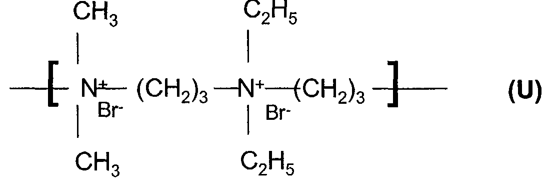 et notamment ceux dont le poids moléculaire, déterminé par chromatographie par perméation de gel, est d'environ 1200;
   - les polymères de type poly(ammonium quaternaire) de la famille (11) avec X⁻ désignant le chlore, et notamment ceux dont la masse moléculaire moyenne en poids est inférieure à 100 000, de préférence inférieure ou égale à 50 000;
(ii) parmi les polymères amphotères :
   - le copolymère chlorure de diméthyldiallylammonium/acide acrylique (80/20) (Merquat 280 de Nalco- dénomination CTFA : Polyquaternium 22) ;
   - le copolymère chlorure de diméthyldiallylammonium/acide acrylique (95/5) (Merquat 295 de Nalco) ;
   - le copolymère de chlorure de méthacrylamidopropyltrimonium, d'acide acrylique et d'acrylate d'éthyle (Merquat 2001 de Nalco-dénomination CTFA : Polyquaternium 47) ;
   - le terpolymère acrylamide/chlorure de diméthyldiallylammonium/acide acrylique (Merquat Plus 3330 de Nalco-dénomination CTFA : Polyquaternium 39).

Lorsqu'ils sont présents dans la composition, la teneur en polymère conditionneur est comprise entre 0,01 et 10% en poids par rapport au poids total de la composition, et de préférence entre 0,05 et 5% en poids par rapport à la même référence.

### Polymères amphiphiles à chaîne hydrophobe

Ainsi, la composition peut comprendre, selon une variante avantageuse de l'invention, au moins un polymère amphiphile comportant au moins une chaîne hydrophobe. Plus spécialement ces polymères amphiphiles, s'ils sont présents sont de type non ioniques, anioniques, cationiques ou amphotères. De préférence ils sont de nature non ionique, anionique ou cationique.

Plus particulièrement, lesdits polymères amphiphiles comprennent, en tant que chaîne hydrophobe, une chaîne hydrocarbonée, saturée ou non, aromatique ou non, linéaire ou ramifiée, en C₆-C₃₀, comprenant éventuellement un ou plusieurs motifs oxyalkylénés (oxyéthylénés et/ou oxypropylénés).

Parmi les polymères amphiphiles cationiques comportant une chaîne hydrophobe on peut trouver des polyuréthannes cationiques ou des copolymères cationiques comprenant des motifs vinyllactame et en particulier vinylpyrrolidone. Encore plus préférentiellement les polymères amphiphiles comportant une chaîne hydrophobe seront de nature nonionique ou anionique.

A titre d'exemples de polymères amphiphiles non ioniques à chaîne hydrophobe, on peut citer entre autres :
(1) les celluloses modifiées par des groupements comportant au moins une chaîne hydrocarbonée, saturée ou non, linéaire ou ramifiée, en C₆-C₃₀, comme les hydroxyéthylcelluloses modifiées par des groupements comportant au moins une chaîne hydrophobe telle que définie auparavant, comme notamment Natrosol Plus Grade 330 CS (alkyles en C₁₆ - commercialisé par la société Aqualon) ; Bermocoll EHM 100 (commercialisé par la société Berol Nobel), Amercell Polymer HM-1500 (hydroxyéthylcellulose modifiée par un groupement polyéthylène glycol (15) éther de nonylphénol - commercialisé par la société Amerchol).
(2) les hydroxypropylguars modifiés par des groupements comportant au moins une chaîne hydrophobe telle que définie, par exemple Jaguar XC-95/3 (chaîne alkyle en C₁₄ - commercialisé par la société Rhodia Chimie) ; Esaflor HM 22 (chaîne alkyle en C₂₂ - commercialisé par la société Lamberti) ; RE210-18 (chaîne alkyle en C₁₄) et RE205-1 (chaîne alkyle en C₂₀) commercialisés par la société Rhodia Chimie.
(3) les copolymères de vinylpyrrolidone et de monomères hydrophobes à chaîne hydrophobe telle que définie auparavant comme par exemple Antaron ou Ganex V216 (copolymères vinylpyrrolidone / hexadécène) ; Antaron ou Ganex V220 (copolymères vinylpyrrolidone / eicosène), commercialisés par la société I.S.P.
(4) les copolymères de (méth)acrylates d'alkyles en C₁-C₆ et de monomères amphiphiles comportant une chaîne hydrophobe.
(5) les copolymères de (méth)acrylates hydrophiles et de monomères hydrophobes comportant au moins une chaîne hydrophobe, tels que par exemple le copolymère méthacrylate de polyéthylèneglycol / méthacrylate de lauryle.
(6) les polymères à squelette aminoplaste éther possédant au moins une chaîne grasse, tels que les composés Pure Thix commercialisés par la société Süd-Chemie.
(7) les polyéthers polyuréthannes, linéaires (structure à blocs), greffés ou en étoile, comportant dans leur chaîne, au moins une séquence hydrophile, généralement polyoxyéthylénée et pouvant comprendre entre 50 et 1000 motifs oxyéthylène environ, et au moins une séquence hydrophobe, qui peut comprendre des groupements aliphatiques seuls, éventuellement combinés à des enchaînements cycloaliphatiques et/ou aromatiques. De préférence, les polyéthers polyuréthanes comportent au moins deux chaînes hydrophobes hydrocarbonées en C₆-C₃₀, séparées par une séquence hydrophile ; les chaînes hydrophobes pouvant être des chaînes pendantes ou des chaînes à l'une ou plusieurs des extrémités de la ou des séquences hydrophiles.
   Les polyéthers polyuréthannes comportent une liaison uréthanne entre les séquences hydrophiles, d'où l'origine du nom. Par extension figurent aussi parmi les polyéthers polyuréthannes dont les séquences hydrophiles sont liées aux séquences lipophiles par d'autres liaisons chimiques.
   Les polyéthers polyuréthannes utilisables selon l'invention sont en particulier ceux décrits dans l'article de G. Fonnum, J. Bakke et Fk. Hansen - Colloid Polym. Sci 271, 380.389 (1993). A titre d'exemples de polyéthers polyuréthannes, on peut citer Nuvis FX 1100 (désignation I.N.C.I. européenne et américaine "Steareth-100/PEG-136/H.M.D.I. Copolymer" commercialisé par la société Servo Delden) ; Rheolate 205, 208 , 204 ou 212 (commercialisés par la société Rheox) ; Elfacos T210 (chaîne alkyle en C₁₂- C₁₄) Elfacos T212 (chaîne alkyle en C₁₈) commercialisés par la société Akzo.

Les polymères amphiphiles anioniques à chaîne hydrophobe, susceptibles d'être mis en oeuvre comportent au moins à titre de chaîne hydrophobe, une chaîne hydrocarbonée, saturée ou non, aromatique ou non, linéaire ou ramifiée, en C₈-C₃₀.

Plus particulièrement les polymères amphiphiles anioniques comportant au moins une chaîne hydrophobe utilisables dans le cadre de la présente invention, réticulés ou non, comprennent au moins un motif hydrophile dérivé d'un ou de plusieurs monomères à insaturation éthylénique portant une fonction acide carboxylique, ou une fonction acide sulfonique, libre ou partiellement ou totalement neutralisée, et au moins un motif hydrophobe dérivé d'un ou de plusieurs monomères à insaturation éthylénique portant une chaîne latérale hydrophobe, et éventuellement au moins un motif de réticulation dérivés d'un ou plusieurs monomères polyinsaturés.

A titre de monomère à insaturation éthylénique portant une fonction acide carboxylique, on peut citer l'acide éthacrylique, l'acide méthacrylique et l'acide acrylique, ou leurs mélanges ; les deux derniers étant préférés.
A titre d'exemples de monomères à insaturation éthylénique portant une chaîne latérale hydrophobe peuvent être cités les esters d'acides carboxyliques insaturés, comme notamment les acides éthacrylique, méthacrylique, acrylique, et d'alcools saturés, linéaires ou ramifiés, en C₈-C₃₀, plus particulièrement en C₁₂-C₂₂, éventuellement oxyalkyléné (de préférence oxyéthyléné). Ils peuvent aussi être des éthers allyliques d'alcools saturés ou non, aromatiques ou non, ramifiés ou non, en C₆-C₃₀, éventuellement oxyalkyléné (de préférence oxyéthyléné), plus particulièrement de formule CH₂=CR'CH₂OBₙR, avec R' représentant H ou CH₃, B représentant le radical éthylèneoxy, n est un entier compris entre 0 et 100, R représente un radical hydrocarboné choisi parmi les radicaux alkyle, arylalkyle, aryle, alkylaryle, cycloalkyle, comportant de 8 à 30 atomes de carbone. Un motif plus particulièrement préféré est tel que R' représente l'hydrogène, n est égal à 10, et R représente un radical stéaryle (C₁₈).
Concernant le monomère réticulant, celui-ci comprend au moins deux doubles liaisons polymérisables non conjuguées l'une avec l'autre. On peut citer à titre d'exemples le phtalate de diallyle, le (méth)acrylate d'allyle, le divinylbenzène, le diméthacrylate de (poly)éthylèneglycol, le méthylène-bis-acrylamide, le polyallylsucrose ou le polyallylpentaérythritol. Des polymères amphiphiles anioniques du type décrit ci-dessus sont décrits et préparés, par exemple dans les brevets US 3915921 et US 4509949 (copolymères d'acide (méth)acrylique et de (méth)acrylates d'alkyles en C₁₀-C₃₀) ou dans le brevet EP 216479 (copolymères d'acide (méth)acrylique et d'éthers allyliques d'alcools gras).

Les polymères amphiphiles comportant au moins un groupement sulfonique, sous forme libre ou partiellement ou totalement neutralisée et au moins une partie hydrophobe sont par exemple décrits dans FR 0016954 et FR 0100328 dont le contenu fait partie intégrante de la présente invention.
On peut citer plus particulièrement parmi eux le copolymère acide acrylamido-2-méthyl-2-propane-sulfonique (AMPS)/n-dodécylacrylamide neutralisé par la soude, le copolymère réticulé par du méthylène-bis-acrylamide constitué de 75% en poids de motifs AMPS neutralisés par NH₃ et de 25% en poids de motifs acrylate de Genapol T-250, le copolymère réticulé par du méthacrylate d'allyle constitué de 90% en poids de motifs AMPS neutralisés par NH₃ et de 10% en poids de motifs méthacrylate de Genapol T-250, ou le copolymère réticulé par du méthacrylate d'allyle constitué de 80% en poids de motifs AMPS neutralisés par NH₃ et de 20% en poids de motifs méthacrylate de Genapol T-250.

On peut citer à titre d'exemples de polymères préférés, Carbopol ETD-2020 (copolymère acide acrylique/méthacrylate d'alkyle en C10-C30, réticulé - commercialisé par la société Noveon) ; Carbopol 1382, Pemulen TR1, Pemulen TR2 (copolymères acide acrylique/acrylate d'alkyle en C₁₀-C₃₀, réticulés - commercialisés par la société Noveon). le copolymère acide méthacrylique/acrylate d'éthyle/méthacrylate de stéaryle oxyéthyléné (55/35/10); le copolymère acide (méth)acrylique/acrylate d'éthyle/méthacrylate de béhényle oxyéthyléné 25 OE (Aculyn 28 commercialisé par Rohm & Haas) et le copolymère réticulé acide méthacrylique/acrylate d'éthyle/stéareth-10 allyl éther.

Si ces polymères amphiphiles sont présents, leur teneur représente de 0,005 à 20 % en poids par rapport au poids total de la composition selon l'invention, de préférence de 0,1 à 10 % en poids par rapport à la même référence.

### Agents épaississants

A titre d'agent épaississant, la composition peut aussi comprendre au moins un polymère hydrosoluble épaississant dépourvu de chaîne hydrophobe. Les polymères convenables peuvent être d'origine naturelle, ou être des polymères synthétiques, et sont avantageusement choisis parmi ceux utilisés classiquement dans le domaine cosmétique. En outre, ces polymères ne présentent pas de chaîne hydrophobe, c'est-à-dire de chaîne hydrocarbonée, saturée ou non, aromatique ou non, linéaire ou ramifiée, en C₈-C₃₀, comprenant éventuellement un ou plusieurs motifs oxyalkylénés (oxyéthylénés et/ou oxypropylénés). Les polymères hydrosolubles épaississants sont donc différents des polymères amphiphiles qui viennent d'être décrits.

Comme exemples de polymères synthétiques, on peut citer, la polyvinylpyrrolidone, l'acide polyacrylique, le polyacrylamide, l'acide poly-2-acrylamidopropanesulfonique non réticulé (Simugel EG de la société SEPPIC), l'acide poly-2-acrylamido-2-méthylpropane sulfonique réticulé, libre ou partiellement neutralisé par l'ammoniaque (Hostacerin AMPS de Clariant), des mélanges d'acide poly-2-acrylamido-2-méthylpropane sulfonique non réticulé avec des éthers d'hydroxyalkylcellulose ou avec des poly(oxyde d'éthylène) tel qu'ils sont décrits dans le brevet US 4540510 ; des mélanges d'acide poly(méth)acrylamido-alkyl(C₁-C₄)-sulfonique, de préférence réticulé, avec un copolymère réticulé de l'anhydride maléique et d'un alkyl(C₁-C₅)vinyléther (Hostacerin AMPS / Stabileze QM de la société ISF).

Les polymères épaississants d'origine naturelle utilisables selon la présente invention, sont de préférence des polymères comportant au moins un motif sucre, comme les gommes de guar non ioniques, modifiées ou non par des groupements hydroxyalkyle en C₁-C₆ ; les gommes de biopolysaccharides d'origine microbienne telles que les gommes de Scléroglucane ou de Xanthane ; les gommes issues d'exudats végétaux telles que les gommes Arabique, Ghatti, Karaya, Tragacanthe, Carraghénanne, Agar et Caroube ; les pectines ; les alginates ; les amidons ; les hydroxyalkyl(C₁-C₆)celluloses et carboxyalkyl(C₁-C₆)celluloses.

Ici, "motif sucre" désigne une portion monosaccharidique (i.e. monosaccharide ou oside ou sucre simple), une portion oligosaccharidique (chaînes courtes formées de l'enchaînement d'unités monosaccharidiques, éventuellement différentes) ou une portion polysaccharidique [longues chaînes constituées d'unités monosaccharidiques, éventuellement différentes, i.e. polyholosides ou polyosides]. Les unités saccharidiques peuvent être en outre substituées par des radicaux alkyle, ou hydroxyalkyle, ou alcoxy, ou acyloxy, ou carboxyle, les radicaux alkyle comportant de 1 à 4 atomes de carbone.

A titre d'exemples de gommes de guar non ioniques non modifiées, on peut citer entre autres Guargel D/15 (Noveon) ; Vidogum GH 175 (Unipectine), Meypro-Guar 50 et Jaguar C (Meyhall/Rhodia Chimie) ; et à titre de gommes de guar non ioniques modifiées, Jaguar HP8, HP60, HP120, DC 293, HP 105 (Meyhall/Rhodia Chimie) ; Galactasol 4H4FD2 (Aqualon).

Les gommes de biopolysaccharides d'origine microbienne, végétale sont bien connues de l'homme de l'art et décrites notamment dans l'ouvrage de Robert L. Davidson intitulé "Handbook of Water soluble gums and resins" édité chez Mc Graw Hill Book Company (1980).
Parmi ces gommes, citons les scléroglucanes comme notamment Actigum CS de Sanofi Bio Industries ; Amigel de Alban Muller International, ainsi que les scléroglucanes traités au glyoxal décrits dans FR 2633940) ; les gommes xanthanes comme Keltrol, Keltrol T, Keltrol Tf, Keltrol Bt, Keltrol Rd, Keltrol Cg (Nutrasweet Kelco), Rhodicare S, Rhodicare H (Rhodia Chimie) ; les dérivés d'amidon comme Primogel (Avebe) ; les hydroxyéthylcelluloses telles que Cellosize QP3L, QP4400H, QP30000H, HEC30000A, Polymer PCG10 (Amerchol), Natrosol 250HHR, 250MR, 250M, 250HHXR, 250HHX, 250HR, HX (Hercules), Tylose H1000 (Hoechst) ; les hydroxypropylcelluloses comme Klucel EF, H, LHF, MF, G (Aqualon) ; les carboxyméthylcelluloses comme Blanose 7M8/SF, raffinée 7M, 7LF, 7MF, 9M31F, 12M31XP, 12M31P, 9M31XF, 7H, 7M31, 7H3SXF (Aqualon), Aquasorb A500 (Hercules), Ambergum 1221 (Hercules), Cellogen HP810A, HP6HS9 (Montello), Primellose (Avebe).

La composition peut de plus comprendre à titre d'agent épaississant, en remplacement ou en association avec au moins un polymère hydrosoluble dépourvu de chaîne hydrophobe, au moins un alkylamide d'acide carboxylique comprenant de 6 à 30 atomes de carbone, linéaire ou non, saturé ou non, et portant éventuellement un ou plusieurs groupements hydroxyle.
Par ailleurs, l'azote du groupement amide peut être mono- ou di-substitué. Il est de préférence monosubstitué.
Enfin, selon un mode de réalisation particulier, l'amide comprend 1 à 20 motifs oxyalkylénés (oxyéthylénés et/ou oxypropylénés), de préférence 1 à 10 motifs et de plus particulièrement 1 à 5 motifs. De préférence, l'amide porte des motifs oxyéthylénés.

La teneur en agents épaississants, s'ils sont présents, représente de 0,005 à 20 % en poids, par rapport au poids total de la composition, de préférence de 0,1 à 10 % en poids, par rapport à la même référence.

### Agents tensioactifs

La composition de décoloration anhydre peut de plus comprendre un ou plusieurs tensioactifs, choisis parmi les tensioactifs anioniques, non ioniques, cationiques, amphotères, zwitterioniques ou leurs mélanges.

A titre d'exemples non limitatifs de tensioactifs anioniques utilisables, seuls ou mélanges, dans le cadre de la présente invention, on peut citer notamment les sels (en particulier sels alcalins, notamment de sodium, magnésium, sels d'ammonium, d'amines, d'aminoalcools) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄) polyglycosides carboxyliques tels que les alkylglycoside citrates, tartrates et sulfosuccinates, les alkylsulfosuccinamates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle. Parmi les tensioactifs anioniques encore utilisables, on peut également citer les sels d'acides gras (notamment en C₆-C₂₄) tels que les sels des acides oléique, ricinoléique, et palmitique, des acides d'huile de coprah ou d'huile de coprah hydrogénée, et notamment de préférence les sels de sodium, calcium ou magnésium de l'acide stéarique; les acyl-lactylates dont le radical acyle comporte 8 à 20 atomes de carbone. On peut également utiliser les acides d'alkyl D galactoside uroniques et leurs sels, les acides alkyl(C₆-C₂₄) éther carboxyliques polyoxyalkylénés, les acides alkyl(C₆-C₂₄) aryl éther carboxyliques polyoxyalkylénés, les acides alkyl(C₆-C₂₄)amido éther carboxyliques polyoxyalkylénés et leurs sels, en particulier ceux comportant de 2 à 50 groupements oxyde d'alkylène en particulier d'éthylène, et leurs mélanges.

Sans vouloir s'y limiter, les tensioactifs non ioniques peuvent être choisis, seuls ou en mélange, parmi les alcools, les alpha-diols, les alkylphénols polyéthoxylés et/ou polypropoxylés, ayant une chaîne comportant par exemple 6 à 24 atomes de carbone, le nombre de groupements oxyde d'éthylène et/ou oxyde de propylène pouvant aller notamment de 1 à 50. On peut également citer les copolymères d'oxyde d'éthylène et de propylène, les condensats d'oxyde d'éthylène et de propylène sur des alcools gras (notamment en C₆-C₂₄) ; les amides gras (notamment en C₆-C₂₄) polyéthoxylés ayant de préférence de 2 à 30 moles d'oxyde d'éthylène, les alcools gras (notamment en C₆-C₂₄) mono- ou poly-glycérolés comportant en moyenne 1 à 30 groupements glycérol et les amides gras (notamment en C₆-C₂₄) polyglycérolés comportant en moyenne 1 à 5 groupements glycérol et en particulier 1,5 à 4 ; les esters d'acides gras (notamment en C₆-C₂₄) du sorbitan oxyéthylénés ayant par exemple de 2 à 30 moles d'oxyde d'éthylène ; les esters d'acides gras (notamment en C₆-C₂₄) du sucrose, les esters d'acides gras (notamment en C₆-C₂₄) du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine.

A titre d'illustration, les agents tensioactifs amphotères ou zwitterioniques peuvent être notamment des dérivés d'amines secondaires ou tertiaires aliphatiques, dans lesquels le radical aliphatique est une chaîne linéaire ou ramifiée, en C₈-C₁₈, et contenant au moins un groupe anionique du type carboxylate, sulfonate, sulfate, phosphate ou phosphonate ; des alkyl(C₈-C₂₀)bétaïnes, les alkyl(C₈-C₂₀)sulfobétaïnes, les alkyl(C₈-C₂₀) amidoalkyl (C₁-C₆)bétaïnes ou les alkyl(C₈-C₂₀)amidoalkyl(C₁-C₆)sulfobétaïnes. Conviennent aussi les ampho- carboxyglycinates les amphocarboxypropionates, classés dans le dictionnaire CTFA, 5ème édition, 1993, notamment sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauro-amphodipropionate, Disodium Caprylampho-dipropionate, Disodium Capryloampho-dipropionate, Lauroamphodipropionic acid, Cocoamphodipropionic acid. A titre d'exemples on peut citer le Cocoamphodiacétate (Miranol® C2M concentré de Rhodia Chimie).

Parmi les tensioactifs cationiques on peut citer à titre purement indicatif, les sels d'amines grasses primaires, secondaires ou tertiaires, éventuellement polyoxyalkylénées; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium; les dérivés d'imidazoline ; ou les oxydes d'amines à caractère cationique.

De préférence, s'ils sont présents, les tensioactifs sont choisis parmi les composés anioniques et/ou non ioniques.

Au cas où un ou plusieurs tensioactifs seraient présents dans la composition, leur teneur est comprise entre 0,01 et 40% en poids par rapport au poids total de la composition, de préférence entre 0,1 et 30% en poids par apport à la même référence.

### Autres additifs

La composition peut comprendre de plus tout autre type d'additifs utilisés tels que des agents réducteurs (comme par exemple l'acide thioglycolique ou ses sels, l'acide thiolactique ou ses sels, la cystéine, la cystéamine), des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, etc....
La composition peut de plus comprendre des charges minérales ou organiques (comme par exemple de la silice pyrogénée à caractère hydrophobe), des liants, des lubrifiants, des agents antimousse, des agents colorants, des agents matifiants, des parfums, etc.

### pH de la composition

Notons que le pH de la composition selon l'invention est avantageusement compris entre 4 et 11, plus particulièrement entre 8 et 11.

L'ajustement du pH de la composition peut être réalisé au moyen d'agent alcalinisant ou acidifiant.
A titre d'exemples d'agents acidifiants susceptibles d'être utilisés, on peut citer les acides minéraux ou organiques comme l'acide chlorhydrique, l'acide phosphorique, l'acide orthophosphorique, l'acide acétique, l'acide tartrique, l'acide citrique, l'acide lactique, l'acide borique et les acides sulfoniques.
Les agents alcalinisants peuvent, eux, être notamment choisis parmi l'ammoniaque, les carbonates alcalins ou d'ammonium, les alcanolamines telles que les mono-, di- et triéthanolamines ainsi que leurs dérivés, les hydroxyalkylamines, les éthylène-diamines oxyéthylénées et/ou oxypropylénées, les hydroxydes de sodium ou de potassium et les composés répondant à la formule suivante : 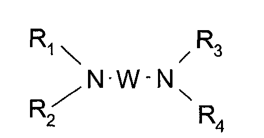 dans laquelle W est un reste propylène éventuellement substitué par un groupement hydroxyle ou un radical alkyle en C₁-C₆ ; R₁, R₂, R₃ et R₄, identiques ou différents, représentent un atome d'hydrogène, un radical alkyle en C₁-C₆ éventuellement porteur d'au moins un radical hydroxyle.

Habituellement, la composition présente une teneur en agent acidifiant ou alcalinisant, s'il est (sont) présent(s), comprise entre 0,01 et 30% en poids par rapport au poids total de la composition.

La composition selon l'invention peut se trouver sous des formes diverses, telles que des liquides, des crèmes, des gels, des pâtes, ou sous toute autre forme appropriée.

Un autre objet de la présente invention est constitué par une composition prête à l'emploi comprenant la composition qui vient d'être détaillée et une composition oxydante, comprenant dans un milieu approprié pour la teinture, au moins un agent oxydant.

Dans une variante de l'invention, cette composition prête à l'emploi peut être obtenue par mélange extemporané avant applicationn, d'une composition telle que décrite ci-dessus et comprenant au moins une base d'oxydation et/ou au moins un colorant direct avec une composition comprenant au moins un agent oxydant.

De manière avantageuse, l'agent oxydant est choisi parmi le peroxyde d'hydrogène, le peroxyde d'urée, les bromates de métaux alcalins, les persels tels que les perborates, percarbonates et persulfates, les peracides, les enzymes telles que les peroxydases et les oxydo-réductases à deux ou quatre électrons, ou leurs mélanges.

La teneur en agent(s) oxydant(s) représente plus particulièrement de 0,1 à 30% en poids par rapport au poids de la composition oxydante, de préférence de 0,5 à 20% en poids par rapport à la même référence.

Par ailleurs, conformément à un mode de réalisation particulier de l'invention, la teneur en agent oxydant est comprise entre 5 et 20 % en poids par rapport au poids de la composition prête à l'emploi.

Le milieu de la composition oxydante est un milieu approprié pour la teinture et peut être choisi parmi ceux listés dans le cadre de la composition selon l'invention. On pourra donc s'y référer.

Il est à noter que le pH de la composition prête à l'emploi est avantageusement compris entre 4 et 12, plus particulièrement entre 7 et 11,5, et de préférence entre 8 et 11.

L'invention a de même pour objet un procédé de coloration de fibres kératiniques, comprenant successivement les étapes suivantes :
a) on applique sur les fibres kératiniques une composition selon l'invention, comprenant au moins une base d'oxydation et/ou au moins un colorant direct, et au moins un composé de formule (I) telle que définie précédemment, et éventuellement une composition oxydante, celles-ci étant mélangées extemporanément avant l'emploi ou appliquées successivement ;
b) on laisse reposer pendant un temps suffisant pour obtenir la coloration recherchée ;
c) on rince les fibres kératiniques pour en éliminer la composition selon l'invention et éventuellement la composition oxydante ;
d) on lave éventuellement les fibres kératiniques, on les rince ;
e) on les sèche éventuellement.

Il est précisé que le procédé selon l'invention est tout particulièrement adapté pour la coloration de fibres kératiniques humaines, et de préférence les cheveux, les cils, les sourcils, la barbe, la moustache.

Selon une première variante de l'invention, le procédé est mis en oeuvre avec une composition comprenant au moins un colorant direct et au moins un composé de formule (1) en l'absence de composition oxydante. Notons que selon cette variante, la composition selon l'invention ne comprend pas de base d'oxydation ni de coupleur.

Selon une deuxième variante du procédé, ce dernier est mis en oeuvre avec une composition comprenant au moins une base d'oxydation, éventuellement associée à au moins un coupleur, et/ou au moins un colorant direct, et comprenant au moins un composé de formule (I) en présence d'une composition oxydante.

Selon cette deuxième variante, le procédé comporte une étape préliminaire consistant à stocker sous forme séparée, d'une part, la composition selon l'invention et, d'autre part, la composition oxydante, puis à procéder à leur mélange au moment de l'emploi avant d'appliquer ce mélange sur les fibres kératiniques pendant un temps suffisant pour développer la coloration désirée, après quoi on les rince, on les lave éventuellement au shampooing, on les rince à nouveau et éventuellement, on les sèche.

Les éléments constitutifs et leurs teneurs respectives ont été définies précédemment dans la description et l'on pourra s'y référer.

De préférence, la composition oxydante est utilisée en quantité telle que la teneur en agent oxydant dans le mélange comprenant la composition selon l'invention et la composition oxydante (composition prête à l'emploi), est comprise entre 5 et 20 % en poids par rapport au poids de la composition prête à l'emploi.

Quelle que soit la variante du procédé retenue, la température nécessaire au développement de la coloration est généralement comprise entre la température ambiante (15 à 25°C) et 80°C et plus particulièrement entre 15 et 40 °C.

Habituellement, le temps de pause varie entre 1 et 60 minutes, plus préférentiellement entre 10 et 50 minutes.

Enfin, un dernier objet de l'invention est constitué par un dispositif pour la coloration de fibres kératiniques, comprenant au moins deux compartiments, l'un d'eux comprenant une composition selon l'invention, l'autre comprenant une composition oxydante comprenant dans un milieu approprié pour la teinture, au moins un agent oxydant.

Tout ce qui a été indiqué auparavant concernant la nature et les teneurs des divers éléments constitutifs de la composition selon l'invention ainsi que de la composition oxydante, reste valable et ne sera pas repris dans cette partie de la description.

Des exemples concrets mais non limitatifs de l'invention vont maintenant être présentés.

### EXEMPLE 1

On prépare la formulation suivante, se trouvant sous forme liquide :

| | |
|---|---|
| Alcool oléique | 4 % |
| Alcool oléique polyglycérolé (2 moles) [Chimexane NB- commercialisé par Chimex] | 8 % |
| Alcool oléique polyglycérolé (4 moles) [Chimexane NC - commercialisé par Chimex] | 5 % |
| Lauryl aminosuccinamate de diéthylaminopropyle à 55 % dans l'eau [Chimexane HB- commercialisé par Chimex] | 2 % |
| Acide oléique | 5 % |
| Monoéthanolamide d'acide d'alkyl (C13/C15) éther carboxylique (2OE) [AminolA15 - commercialisé par Chemy] | 12 % |
| Amine oléique oxyéthylénée (2OE) [Rhodameen 02 - commercialisée par Rhodia Chimie] | 5 % |
| Ethanol | 9 % |
| Propylène glycol | 5 % |
| Butoxydiglycol | 10 % |
| 2-hydroxyéthyliminodi(acétate) de disodium en solution aqueuse à 28 % [proposé par Dow Chemical] | 0,96 % |
| | |
| 1,3-dihydroxybenzène (résorcinol) | 0,085 % |
| Paraphénylènediamine | 0,27 % |
| 5-N-(β-hydroxyéthyl)amino-2-méthyl-phénol | 0,16 % |
| 2-méthyl-5-aminophénol | 1,12 % |
| Para-aminophénol | 0,2 % |
| 6-hydroxy indole | 0,045 % |
| | |
| Antioxydant | qs |
| Réducteur | qs |
| Parfum | qs |
| Ammoniaque (20% NH₃) | 10,2 % |
| | |
| Eau | qsp 100% |

Les pourcentages sont exprimés en poids.

Au moment de l'emploi, cette composition est mélangée poids par poids avec une composition oxydante titrant 6 % en peroxyde d'hydrogène et le ménage obtenu est appliqué 30 minutes sur cheveux puis rincé.
On obtient une nuance peu sélective.

### EXEMPLE 2

On prépare la formulation suivante, se trouvant sous forme de crème :

| | |
|---|---|
| Alcool cétylstéarylique | 13 % |
| Alcool laurique oxyéthyléné (12OE) | 8 % |
| Alcool décylique oxyéthyléné (3OE) à 90 % dans l'eau [Empilan KA 2,5/90FL - commercialisé par HUNSTMANN] | 6 % |
| Alcool oléocétylique oxyéthyléné (30OE) | 4 % |
| Acide laurique | 5 % |
| Monoéthanolamine | 2 % |
| Propylène glycol | 8 % |
| Polycondensat tétraméthyl héxaméthylène diamine / dichloro 1,3-propylène à 60 % dans l'eau [Mexomere PO (G1847) - commercialisé par CHIMEX] | 1 % |
| Copolymère chlorure de diméthyl diallylammonium / acide acrylique (80/20) à 40,5 % dans l'eau [Merquat 280 - commercialisé par Nalco] | 2 % |
| Distéarate de glycol | 4 % |
| Silice pyrogénée à caractère hydrophobe | 2 % |
| Acide polyacrylique réticulé [Carbopol 980 - commercialisé par Noveon] | 0,6 % |
| | |
| Sel trisodique de l'acide méthylglycinediacétique en solution aqueuse à 40% [Trilon M liquide - commercialisé par BASF] | 1 % |
| | |
| 1,3-dihydroxybenzène (résorcinol) | 0,67 % |
| Paraphénylènediamine | 0,88 % |
| 5-N-(β-hydroxyéthyl)amino-2-méthyl-phénol | 0,055 % |
| 2-méthyl-1,3-dihydroxy benzène | 0,11 % |
| Para-aminophénol | 0,27 % |
| 4-(méthylamino)phénol hémisulfate | 0,26 % |
| 1-hydroxy-3-amino benzene | 0,16 % |
| | |
| Antioxydant | qs |
| Réducteur | qs |
| Parfum | qs |
| Ammoniaque (20% NH₃) | 11,1 % |
| | |
| Eau | qsp 100% |

Les pourcentages sont exprimés en poids.

Au moment de l'emploi, cette composition est mélangée avec une fois et demi son poids d'une composition oxydante titrant 6 % en peroxyde d'hydrogène et le mélange obtenu est appliqué sur cheveux pendant 30 minutes puis rincé.
On obtient une nuance peu sélective.

## Revendications

**1.** Composition destinée à la coloration des fibres kératiniques, comprenant dans un milieu approprié pour la teinture :
a) au moins une base d'oxydation et/ou au moins un colorant direct ;
b) au moins un composé de formule générale (I) suivante :
R-N-(CH(R')CO₂X)₂ (I)
dans laquelle :
- R représente un atome d'hydrogène ou un groupe CH(CO₂X)-(CH₂)₂CO₂X, CH₂-CH₂-OH, CH(CH₃)-CO₂X ou (CH₂)₂-N(COR")-CH₂-CO₂X;
- R" représente un groupe alkyle linéaire ou ramifié et comportant de 1 à 30 atomes de carbone ou cyclique comportant de 3 à 30 atomes de carbone ;
- R' représente un groupe CH₂CO₂X si R représente un atome d'hydrogène ou R' représente un atome d'hydrogène si R est différent d'un atome d'hydrogène ; et
- X représente un atome d'hydrogène ou un cation monovalent ou divalent choisi parmi ceux issus des métaux alcalins, alcalino-terreux, des métaux de transition, des amines organiques ou d'ions ammonium.

**2.** Composition selon la revendication 1, dans laquelle le ou les composés de formule (I) sont choisis parmi l'acide méthylglycinediacétique, l'acide 2-hydroxyéthyliminodiacétique, l'acide N-lauroyl-N,N',N'-triacétique éthylènediamine, l'acide iminodisuccinique, l'acide N,N-dicarboxyméthyl L-glutamique et leurs sels correspondants, ainsi que leurs mélanges.

**3.** Composition selon la revendication 1 ou 2, dans laquelle le ou les composés de formule (I) sont choisis parmi l'acide méthylglycinediacétique, l'acide N-lauroyl-N,N',N'-triacétique éthylènediamine, l'acide N,N-dicarboxyméthyl L-glutamique et leurs sels correspondants, ainsi que leurs mélanges.

**3.** Composition selon l'une des revendications 1 ou 2, dans laquelle le ou les composés de formule (I) est l'acide méthylglycinediacétique, éventuellement sous forme de sel.

**4.** Composition selon l'une quelconque des revendications précédentes, **caractérisée en ce que** la teneur en composé de formule (I) est comprise entre 0,001 et 10 % en poids par rapport au poids total de la composition, de préférence entre 0,001 et 5 % en poids par rapport à la même référence.

**5.** Composition selon l'une quelconque des revendications précédentes, **caractérisée en ce que** la ou les bases d'oxydation sont choisies parmi les paraphénylènediamines, les bis-phénylalkylènediamines, les para-aminophénols, les orthoamino-phénols et les bases hétérocycliques et leurs sels d'addition avec un acide ou avec un agent alcalin, seules ou en mélanges.

**6.** Composition selon la revendication précédente, **caractérisée en ce que** la teneur en base(s) d'oxydation représente de 0,0005 à 12 % en poids du poids total de la composition, et de préférence de 0,005 à 6 % en poids par rapport à la même référence.

**7.** Composition selon l'une quelconque des revendications précédentes, **caractérisée en ce qu'**elle comprend au moins un coupleur.

**8.** Composition selon la revendication précédente, **caractérisée en ce que** le coupleur est choisi parmi les métaphénylènediamines, les méta-aminophénols, les métadiphénols et les coupleurs hétérocycliques et leurs sels d'addition avec un acide ou avec un agent alcalin.

**9.** Composition selon l'une des revendications 7 ou 8, **caractérisée en ce que** la teneur en coupleur(s) représente de 0,0001 à 10 % en poids du poids total de la composition, de préférence de 0,005 à 5 % en poids par rapport à la même référence.

**10.** Composition selon l'une quelconque des revendications précédentes, **caractérisée en ce que** le ou les colorants directs sont choisis parmi les colorants benzéniques nitrés, les colorants azoïques, anthraquinoniques, naphtoquinoniques, benzoquinoniques, phénotiaziniques, indigoïdes, xanthéniques, phénanthridiniques, phtalocyanines, ceux dérivés du triarylméthane, seuls ou en mélanges.

**11.** Composition selon la revendication précédente, **caractérisée en ce que** le ou les colorants directs représentent de 0,0005 à 12 % en poids par rapport au poids total de la composition, de préférence de 0,005 à 6 % en poids par rapport à la même référence.

**12.** Composition selon l'une quelconque des revendications précédentes, **caractérisée en ce qu'**elle comprend un polymère conditionneur cationique ou amphotère, ou leurs mélanges.

**13.** Composition selon la revendication précédente, **caractérisée en ce que** la teneur en polymère conditionneur est comprise entre 0,01 et 10% en poids par rapport au poids total de la composition, et de préférence entre 0,05 et 5% en poids par rapport à la même référence.

**14.** Composition selon l'une quelconque des revendications précédentes, **caractérisée en ce qu'**elle comprend au moins un tensioactif non ionique, anionique, cationique, amphotère ou zwittérionique, ou leurs mélanges.

**15.** Composition selon la revendication précédente, **caractérisé en ce que** la teneur en tensioactif est comprise entre 0,01 et 40% en poids par rapport au poids total de la composition, de préférence entre 0,1 et 30% en poids par apport à la même référence.

**16.** Composition selon l'une quelconque des revendications précédentes, **caractérisée en ce qu'**elle comprend au moins un polymère amphiphile non ionique, anionique, cationique ou amphotère, comportant une chaîne hydrophobe.

**17.** Composition selon la revendication précédente, **caractérisée en ce que** la teneur en polymère(s) amphiphile(s) représente 0,005 à 20 % en poids par rapport au poids total de la composition, de préférence, de 0,1 à 10 % en poids par rapport à la même référence.

**18.** Composition selon l'une quelconque des revendications précédentes, **caractérisée en ce qu'**elle comprend au moins un agent épaississant.

**19.** Composition selon la revendication précédente, **caractérisée en ce que** la teneur en agent épaississant est comprise entre 0,05 à 20 % en poids par rapport au poids total de la composition, de préférence, de 0,1 à 10 % en poids par rapport à la même référence.

**20.** Composition selon l'une quelconque des revendications précédentes, **caractérisée en ce qu'**elle comprend au moins un agent acidifiant ou alcalinisant.

**21.** Composition selon la revendication précédente, **caractérisée en ce que** la teneur en agent acidifiant ou alcalinisant représente de 0,01 à 30% en poids par rapport au poids total de la composition.

**22.** Composition prête à l'emploi comprenant la composition selon l'une quelconque des revendications précédentes et comprenant au moins un agent oxydant.

**23.** Composition prête à l'emploi selon la revendication précédente, **caractérisée en ce que** l'agent oxydant est choisi parmi le peroxyde d'hydrogène, le peroxyde d'urée, les bromates de métaux alcalins, les persels tels que les perborates, percarbonates et persulfates, les peracides, les enzymes telles que les peroxydases et les oxydo-réductases à deux ou quatre électrons, ou leurs mélanges.

**24.** Composition prête à l'emploi selon l'une des revendications 22 ou 23, **caractérisée en ce que** la teneur en agent(s) oxydant(s) représente 0,1 à 30% en poids par rapport au poids de la composition oxydante, de préférence 0,5 à 20% en poids par rapport à la même référence.

**25.** Procédé de coloration de fibres kératiniques, comprenant successivement les étapes suivantes :
a) on applique sur les fibres kératiniques une composition selon l'une quelconque des revendications 1 à 21 et éventuellement une composition oxydante, celles-ci étant mélangées extemporanément avant l'emploi ou appliquées successivement ;
b) on laisse reposer pendant un temps suffisant pour obtenir la coloration recherchée
c) on rince les fibres kératiniques pour en éliminer la composition selon l'invention et éventuellement la composition oxydante ;
d) on lave éventuellement les fibres kératiniques, on les rince et éventuellement,
e) on les sèche éventuellement.

**26.** Dispositif pour la coloration de fibres kératiniques, comprenant au moins deux compartiments, l'un d'eux comprenant une composition selon l'une des revendications 1 à 21, l'autre comprenant une composition oxydante comprenant dans un milieu approprié pour la teinture, au moins un agent oxydant.
